# EUROPÄISCHE PATENTSCHRIFT

(11) **EP 0 988 872 B1**
(45) Veröffentlichungstag und Bekanntmachung des Hinweises auf die Patenterteilung: **01.12.2004**
(21) Anmeldenummer: 98117863.5
(22) Anmeldetag: 21.09.1998
(51) Int. Cl.: A61N 1/16

(54) **Steckergenerator-Vorrichtung**
Plug device for generating bio-positive waves
Prise de courant mâle générant des ondes bio-positives

(43) Veröffentlichungstag der Anmeldung: 29.03.2000
(73) Patentinhaber: Hess & Volk GmbH, 96253 Untersiemau (DE)
(72) Erfinder: Volk, Hellmut, 96450 Coburg (DE); Hess, Walter, 96253 Untersiemau (DE)
(74) Vertreter: Reinhard - Skuhra - Weise & Partner

(56) Entgegenhaltungen:
- EP-A- 0 090 774
- WO-A-92/05832
- WO-A-95/05128
- DE-A- 4 103 297
- DE-A- 4 141 501
- DE-U- 29 502 054
- US-A- 4 727 222
- US-A- 5 486 648

## Beschreibung

Die vorliegende Erfindung betrifft einen Abschirm- bzw. Informationsstecker als ohne eigene Stromenergie wirksames Gerät zum Abstrahlen einer biopositiven Wirkenergie auf insbesondere elektrische Leitungen zum Zwecke des Entstörens und/oder Auflösens der von den elektrischen Leitungen oder anderen Leitungen ausgehenden schädlichen Strahlungen.

Der Mensch lebt als feinsinniges, allen möglichen Umwelteinflüssen unterworfenes Wesen in immer zunehmenderem Maße in einer Umgebung, in der es sehr viele Stromleitungen, die mit Wechselstrom gespeist sind, gibt. Es ist bekannt, daß ein mit Wechselstrom durchflossener Leiter ein elektromagnetisches Wechselfeld nach außen abgibt, dessen Wirkung auf den Menschen je nach Intensität durchaus gesundheitsschädlich sein kann. Denn jede elektrische Welle hat eine Polarisation. Dabei weist der Wechselstrom mit seiner Frequenz von 50 Hz, 50 rechtspolarisierte und 50 linkspolarisierte Schwingungen pro Sekunde auf. Dabei werden die 50 linkspolarisierten Schwingungen nach einschlägigen Erfahrungsberichten von Mensch, Tier und Pflanze nicht vertragen, die rechtspolarisierten Schwingungen jedoch schon.

Die der vorliegenden Erfindung zugrunde liegende Problematik besteht also allgemein darin, die Wirkung dieser schädlichen Abstrahlungen auf den Menschen oder auf seine Haustiere zu vermindern. Aus dem deutschen Gebrauchsmuster 29 5020 54 ist ein Abschirm- bzw. Informationsstecker bekannt, der ähnlichen Zwecken dienen soll. Der dort vorgeschlagene Abschirmstecker besitzt eine gewisse Abschirmwirkung, die jedoch zu gering ausgebildet ist. Des weiteren benötigt er noch ein sogenanntes Mineralelement, das als Speicher für Informationen (Schwingungsfrequenzen) vorhanden sein muß, um eine gewisse Wirkung zu gewährleisten.

Als nachteilhaft bei dem obigen bekannten Ansatz hat sich neben den Abschirmwirkungen die Tatsache herausgestellt, daß das Aufstellen und Einbringen eines solchen Minerals nicht in allen Anwendungsfällen zweckdienlich ist, sowie die Abschirmwirkung in unnötiger Weise verteuert.

Die europäische Patentschrift EP 0 090 774 beschreibt eine Störschutzeinrichtung für ein elektrisches Gerät, welche einen Netzstecker mit Filterelementen und ein Kabel mit einer Abschirmung aufweist. Die als Metallgeflecht ausgebildete Abschirmung ist über einen Erdleiter mit dem Erdleiter einer Steckdose kontaktierbar. Die Anordnung dient der Dämpfung von Hochfrequenzstörungen in Richtung Netz und in Richtung des angeschlossenen Geräts.

### VORTEILE DER ERFINDUNG

Die erfindungsgemäße Steckergenerator-Vorrichtung mit den Merkmalen des Anspruchs 1 weist gegenüber dem bekannten Lösungsansatz den Vorteil auf, daß das schädliche elektromagnetische Wechselfeld eines stromdurchflossenen Leiters zuverlässiger abgeschirmt wird, als es bisher möglich war, wobei auf ein Mineral zur Unterstützung der Abschirmwirkung verzichtet werden kann.

Die der vorliegenden Erfindung zugrunde liegende Idee besteht darin, einen oder zwei wechselstromführende Leiter mit einer Wicklung aus wenigstens einem, vorzugsweise in der Mitte gefalteten Leiter zu umgeben, wodurch ein eigenes, sekundäres elektromagnetisches Feld erzeugt wird, das bestimmte, bioenergetisch wirksame Wirkungen auf den Stromkreislauf überträgt. Diese vorteilhafte Wirkung pflanzt sich dann im Leiter in Richtung des elektrischen Verbrauchers im Haushalt oder am Arbeitsplatz oder dergleichen fort. Daher sind der Mensch, Pflanzen oder Tiere nicht mehr der schädlichen Abstrahlung aus allen stromdurchflossenen Leitern und Verbrauchern ausgesetzt.

In den Unteransprüchen finden sich vorteilhafte weiterbildungen und Verbesserungen der in Anspruch 1 angegebenen Steckergenerator-Vorrichtung. Gemäß einer bevorzugten Weiterbildung besteht die abschirmende Wicklung aus einer Doppelwicklung von einem in der Mitte gefalteten Draht, die ihrerseits isoliert oder auch nicht isoliert sein kann. Hierbei wird eine besonders gute Abschirmwirkung erzielt.

Gemäß einer weiteren bevorzugten Weiterbildung liegen die einzelnen Windungen aneinander direkt an, sind äquidistant, und die durch eine Windung beschriebene Ebene liegt im wesentlichen senkrecht zur Richtung des oder der stromdurchflossenen Leiter. Hierbei wird die bioenergetisch wirksame Wirkung auf den Stromkreislauf optimiert.

### ZEICHNUNGEN

Ein Ausführungsbeispiel der Erfindung ist in der Zeichnung dargestellt und wird in der nachfolgenden Beschreibung näher erläutert.

Dabei zeigt:

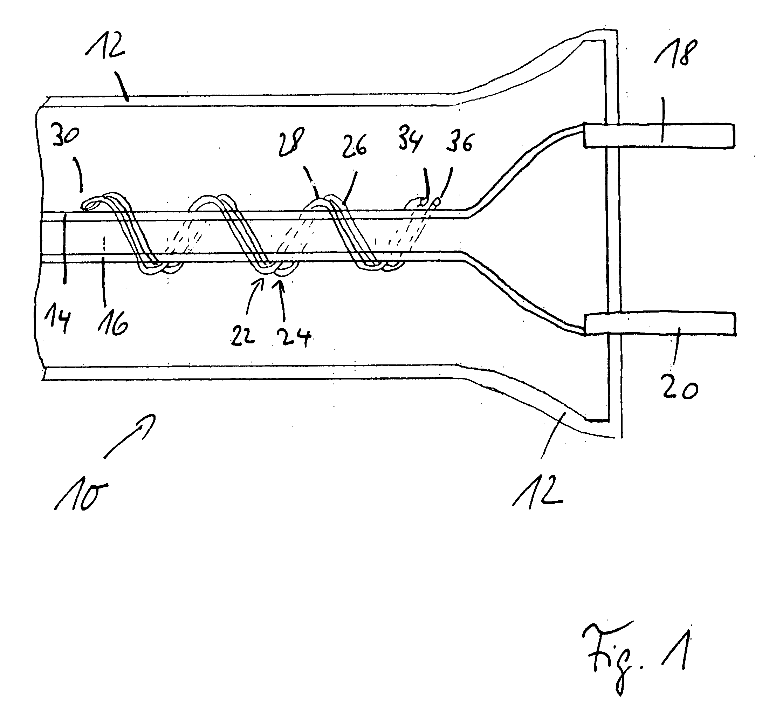

- Fig.1: eine schematische Darstellung der wesentlichen Komponenten der erfindungsgemäßen Steckergenerator-Vorrichtung.

### BESCHREIBUNG DES AUSFÜHRUNGSBEISPIELS

Fig. 1 zeigt eine schematische Darstellung der wesentlichen Komponenten der erfindungsgemäßen Steckergenerator-Vorrichtung.

In Fig. 1 bezeichnet Bezugszeichen 10 die erfindungsgemäße Steckergenerator-Vorrichtung als Ganzes, 12 ihr Gehäuse, 14, 16 Phasenleitungen des Wechselstromnetzes, 18, 20 die zu den Phasenleitungen gehörenden Polstifte, 22, 24 eine Doppelwicklung, die aus einem Draht besteht. Dieser wird in der Mitte gefaltet und dann rechtsdrehend über die beiden elektrischen Phasenleitungen in Richtung der Polstifte in gleichmäßigen Windungen gewickelt.

Die erfindungsgemäße Steckergenerator-Vorrichtung, die als Ganzes mit dem Bezugszeichen 10 bezeichnet ist, enthält im wesentlichen ein übliches Gehäuse 12 als mechanische Abdekkung der in ihrem Inneren befindlichen elektromagnetisch wirksamen Teile. Sie sind zum einen wenigstens zwei elektrische Phasenleitungen 14, 16 zur Kopplung an das Wechselstromnetz. Die Masseleitung ist in der Figur nicht dargestellt, da die Vorrichtung auch in Stromsystemen ohne diese Masseschutzleitung wirksam ist. Sie funktioniert jedoch auch in modernen, dreiphasigen Systemen.

Die Phasenleitungen 14, 16 sind voneinander in üblicher Weise durch eine Kunststoffummantelung isoliert und münden in die zugehörigen Polstifte 18, 20, die zum Einführen in eine herkömmliche Steckdose des Stromnetzes dienen.

Erfindungsgemäß befindet sich nun im Innern des Gehäuses 12 eine Doppelwicklung 22, 24 aus einem durch eine isolierende Ummantelung nach außen hin isolierten Metalldraht 26 bzw. 28. Die Faltung der Wicklung 30 ist geschlossen. In der Zeichnung bestehen die Wicklungen aus jeweils 5 Windungen, die jedoch aus Gründen der Klarheit nicht eng aneinander anliegend gezeichnet sind.

In bevorzugter Weise sind die Wicklungen jedoch eng aneinander anliegend und äquidistant angeordnet, und die einzelnen Drähte dürfen sich nicht berühren, falls auf eine isolierende Ummantelung der Wicklungsdrähte - hier aus Gründen der Klarheit ebenfalls nicht eingezeichnet - verzichtet wird. In besonders bevorzugter Weise verlaufen die Windungen jeweils derart, daß die durch eine vollständige Windung näherungsweise beschriebene Fläche senkrecht zu der von ihr umgebenen Stromleitung verläuft. D.h., die mit dem Netz gekoppelten Stromleitungen durchstoßen diese Ebene quasi senkrecht.

In bevorzugter Weise enthält die Doppelwicklung mindestens acht vollständige Windungen und ist in unmittelbarer Nähe des Endes der bis in die Pole geführten Phasenleitung 14, 16 angeordnet. Die Doppelwicklung sollte so eng sein, daß sie sich nicht ohne Weiteres auf den Phasenleitungen verschieben läßt.

Die so beschriebene Doppelwicklung wirkt als sogenannte Skalar-Antenne, die das elektromagnetische Wechselfeld der Phasenleitungen aufnimmt, ihrerseits ein eigenes, induziertes Wechselfeld aufbaut und dieses wieder in die in ihrem Inneren befindlichen Phasenleitungen einprägt. Diese, von der Doppelwicklung ausgehende Wirkung auf die Phasenleitungen wird auf den gesamten Stromkreislauf in Richtung eines an der Steckergenerator-Vorrichtung angeschlossenen elektrischen Verbrauchers wie z.B. einer Lampe, einem Computer etc. übertragen. Dabei hebt insbesondere die bevorzugte bifilare Wicklung (Doppelwicklung) die eingangs erwähnten linkspolarisierten Schwingungen in ihrer schädlichen Wirkung auf Mensch, Tier und Pflanze in besonderem Maße auf.

Als Folge und besonderer Vorteil der Erfindung ergibt sich ein Lebensraum, der einerseits von den schädlichen Strahlungen abgeschirmt und andererseits über die skalare Wirkung der als Skalarantenne ausgebildeten Doppelwicklung von einer bio-positiven Wirkenergie durchflossen ist.

Obwohl die vorliegende Erfindung anhand eines bevorzugten Ausführungsbeispiels vorstehend beschrieben wurde, ist sie jedoch darauf nicht beschränkt, sondern auf vielfältige Weise modifizierbar.

Insbesondere kann die in Fig. 1 gezeigte, schematisch dargestellte Doppelwicklung ebenso über Treibstoffleitungen, Ölleitungen, Abfüllleitungen für Bier, Wasser, Milch und andere Getränke sowie auf Abfülleitungen von pastenförmigen, rieselfähigen und gekörnten Medien (Stoffen) verwendet werden, um eine bio-positive Wirkenergie in den Innenraum der jeweiligen Leitung abzustrahlen.

Ebenso können die Windungszahl sowie die Abstände der Windungen voneinander je nach Anwendungszweck variieren.

Die vorher beschriebene Wicklung kann auch über einen Metallkern (verschiedene Metalle; kantig, rund oder oval), verschiedene geschliffene Gesteinsarten (kantig, rund oder oval), eine Rohrleitung (verschiedene Rohre mit oder ohne Durchfluß und aus unterschiedlichem Material; kantig, rund oder oval) verwendet werden.

### BEZUGSZEICHENLISTE:

- 10: Steckergenerator
- 12: Gehäuse
- 14, 16: Phasenleitungen
- 18, 20: Polstifte
- 22, 24: Doppelwicklung
- 26, 28: Metalldraht
- 30: Mitte
- 34, 36: Enden

## Patentansprüche

1. Steckergenerator-Vorrichtung (10) zum Abstrahlen einer bio-positiven Wirkenergie mit wenigstens zwei Polstiften (18, 20) zur Kopplung eines elektrischen Verbrauchers über Phasenleitungen (14, 16) an Leitungen einer Steckdose des Wechselstromnetzes,
wobei
die Steckergenerator-Vorrichtung (10) eine Wicklung (22) mit einem oder zwei Leitern um einen Längsabschnitt der mit dem Stromnetz koppelbaren Phasenleitungen (14, 16) in einem Gehäuse (12) der Steckergenerator-Vorrichtung (10) aufweist, und die Windüngsebene der Wicklung (22) im wesentlichen senkrecht zur Richtung der Phasenleitungen (14, 16) verläuft.

2. Steckergenerator-Vorrichtung (10) nach Anspruch 1, **dadurch gekennzeichnet, daß** die Wicklung (22, 24) aus einem in der Mitte gefalteten parallel verlaufenden Leiterstück als Doppelwicklung ausgebildet ist.

3. Steckergenerator-Vorrichtung (10) nach Anspruch 2, **dadurch gekennzeichnet, daß** das Leiterstükk eine elektrische Isolierschicht aufweist.

4. Steckergenerator-Vorrichtung (10) nach einem der vorstehenden Ansprüche 2 oder 3, **dadurch gekennzeichnet, daß** die aus einem in der Mitte gefalteten parallel verlaufenden Leiterstück als Doppelwicklung ausgebildete Wicklung ohne Isolierschicht ausgebildet ist.

5. Steckergenerator-Vorrichtung (10) nach einem der vorstehenden Ansprüche, **dadurch gekennzeichnet, daß** die einzelnen Windungen der Wicklung (22, 24) aneinander anliegen.

6. Steckergenerator-Vorrichtung (10) nach einem der vorstehenden Ansprüche, **dadurch gekennzeichnet, daß** die Länge des von der Wicklung (22, 24) umgebenen Längsabschnittes der Phasenleitungen (14, 16) wenigstens 3 cm beträgt.

## Claims

1. Plug generator apparatus (10) for emitting biopositive real energy having at least two pole pins (18, 20) for the purpose of coupling an electrical load by means of phase lines (14, 16) to lines of a socket of the AC system, the plug generator apparatus (10) having a winding (22) having one or two conductors around a length of the phase lines (14, 16), which can be coupled to the power supply system, in a housing (12) of the plug generator apparatus (10), and the turns plane of the winding (22) running essentially perpendicular to the direction of the phase lines (14, 16).

2. Plug generator apparatus (10) according to Claim 1, **characterized in that** the winding (22, 24) is formed from a parallel conductor piece which is folded in the centre so as to give a dual winding.

3. Plug generator apparatus (10) according to Claim 2, **characterized in that** the conductor piece has an electrical insulating layer.

4. Plug generator apparatus (10) according to one of the preceding Claims 2 or 3, **characterized in that** the winding, which is formed from a parallel conductor piece which is folded in the centre so as to give a dual winding, is formed without an insulating layer.

5. Plug generator apparatus (10) according to one of the preceding claims, **characterized in that** the individual turns of the winding (22, 24) rest on one another.

6. Plug generator apparatus (10) according to one of the preceding claims, **characterized in that** the length of the phase lines (14, 16) which is surrounded by the winding (22, 24) is at least 3 cm long.

## Revendications

1. Dispositif fiche mâle et générateur (10) destiné au rayonnement d'une énergie active biopositive et comportant au moins deux broches polaires (18, 20) pour la connexion d'un consommateur électrique via des fils de phases (14, 16) à des fils d'une fiche femelle du réseau de courant alternatif,
tel que le dispositif fiche mâle et générateur (10) comporte un enroulement (22) avec un ou deux conducteurs autour d'un tronçon longitudinal des fils de phases (14, 16) pouvant être connectés au réseau de courant dans un boîtier (12) du dispositif fiche mâle et générateur (10),
et tel que le plan des spires de l'enroulement (22) s'étend globalement perpendiculairement à la direction des fils de phases (14, 16).

2. Dispositif fiche mâle et générateur (10) selon la revendication 1, **caractérisé par le fait que** l'enroulement (22, 24) est conçu comme un double enroulement à partir d'un morceau de conducteur s'étendant parallèlement et plié au milieu.

3. Dispositif fiche mâle et générateur (10) selon la revendication 2, **caractérisé par le fait que** le morceau de conducteur comporte une couche d'isolation électrique.

4. Dispositif fiche mâle et générateur (10) selon l'une des revendications précédentes 2 ou 3, **caractérisé par le fait que** l'enroulement conçu comme un double enroulement à partir d'un morceau de conducteur s'étendant parallèlement et plié au milieu est conçu sans couche d'isolation.

5. Dispositif fiche mâle et générateur (10) selon l'une des revendications précédentes, **caractérisé par le fait que** les spires individuelles de l'enroulement (22, 24) sont placées les unes contre les autres.

6. Dispositif fiche mâle et générateur (10) selon l'une des revendications précédentes, **caractérisé par le fait que** la longueur du tronçon longitudinal des fils de phases (14, 16) qui est entouré par l'enroulement (22, 24) vaut au moins 3 cm.
